# FASCICULE DE BREVET EUROPEEN

(11) **EP 2 457 612 B1**
(45) Date de publication et mention de la délivrance du brevet: **23.01.2013**
(21) Numéro de dépôt: 11180909.1
(22) Date de dépôt: 12.09.2011
(51) Int. Cl.: A61N 1/05, H01R 11/26

(54) **Ensemble de stimulation/défibrillation du ventricule gauche par voie endocavitaire ou depuis une veine du réseau coronarien**
Instrumentarium zum Stimulieren/Defibrillieren des linken Ventrikels auf endokavitärem Weg oder über eine Koronarvene
Unit for stimulation/defibrillation of the left ventricle via the endocavity or from a vein of the coronary network

(30) Priorité: 29.11.2010 FR 1059847
(43) Date de publication de la demande: 30.05.2012
(73) Titulaire: Sorin CRM SAS, 92140 Clamart (FR)
(72) Inventeur: OLLIVIER, Jean-François, 91190 VILLIERS LE BACLE (FR)
(74) Mandataire: Dupuis-Latour, Dominique

(56) Documents cités:
- EP-A1- 1 557 194
- US-A1- 2001 012 958
- US-A1- 2003 023 296
- US-A1- 2006 064 150
- US-A1- 2009 221 895
- US-A1- 2010 069 983

## Description

L'invention concerne une sonde destinée à la stimulation du ventricule gauche sur un site endocavitaire par application directe d'impulsions sur la paroi interne de ce ventricule, ou indirectement depuis une veine du réseau coronarien.

L'invention se situe dans le contexte général des "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de re-synchronisation ou de défibrillation.

On fera principalement référence dans la présente description à des sondes "de stimulation", c'est-à-dire destinées à la délivrance d'impulsions à basse énergie utilisées pour les thérapies antibradycardiques ou de re-synchronisation. Mais l'invention s'applique également aux sondes de car-dioversion/défibrillation destinées à délivrer au coeur des chocs électriques de haute énergie pour tenter de mettre fin à une tachyarythmie. Sauf indication contraire, les termes génériques de "sonde (ou électrode) de stimulation", ou "de stimulation/défibrillation" viseront tout type de sonde utilisable à ces fins.

Pour la stimulation endocavitaire d'une cavité gauche, avec les techniques proposées jusqu'à présent, il est nécessaire de réaliser dans la paroi septale une ponction de diamètre suffisante pour pouvoir y introduire un cathéter-guide. Ce cathéter-guide est destiné à établir une communication entre ventricule droit et ventricule gauche au travers de la paroi du septum, afin de pouvoir y introduire ensuite la sonde de stimulation endocavitaire gauche.

La demande française 10 53499 du 5 mai 2010, au nom de la Demanderesse, pour un *"Ensemble de stimulation*/*défibrillation endocavitaire du ventricule gauche"* propose de supprimer le cathéter-guide associé à une sonde traversant le septum, en remplaçant cet ensemble par une sonde de type conventionnel vissée sur la paroi du ventricule droit au niveau du septum, et en prolongeant cette sonde par un microcâble transseptal partiellement isolé, poussé dans le ventricule gauche jusqu'à venir en contact contre une cible située dans ce ventricule, par exemple contre la paroi libre de ce dernier.

Dans ce cas, la sonde est utilisée non seulement comme support d'au moins une électrode de détection/stimulation, mais également comme outil de guidage du microcâble au travers de la paroi du septum (pour la ponction qui permettra au microcâble de traverser cette paroi) et au-delà de cette paroi, dans la cavité gauche.

La ponction peut être réalisée par une simple poussée mécanique du microcâble, si la rigidité de celui-ci est suffisante.

Mais le perçage du septum interventriculaire est de préférence assisté par une technique de ponction RF, consistant à appliquer localement une énergie radiofréquence (RF) produite par un générateur électronique approprié afin de créer une ouverture de très faible dimension dans le tissu de la paroi septale. La ponction est obtenue en combinant d'une part la fonction, obtenue par la sonde à vis, de guidage du microcâble, avec d'autre part l'application d'une énergie RF à ce microcâble pour le faire pénétrer progressivement dans le septum et minimiser ainsi considérablement l'effort axial à transmettre à l'extrémité du microcâble pendant cette étape. En effet, en l'absence d'énergie RF appliquée un microcâble trop fin ne pourrait pas pénétrer dans les tissus et s'arc-bouterait au point de contact avec la paroi du septum. On notera par ailleurs que la technique de ponction RF assure une cautérisation des tissus traversés, donc empêche les saignements.

Après perçage, le microcâble est poussé au-delà du septum, dorénavant traversé de part en part, de manière à laisser émerger dans le volume intérieur du ventricule gauche, au-delà de la partie intermédiaire enserrée dans le septum, une partie libre dont la longueur peut aller jusqu'à 120 mm environ. Cette partie libre émergeante du microcâble est totalement ou partiellement dénudée et constitue une partie active susceptible de venir en contact en un ou plusieurs points avec la paroi de la cavité gauche.

Une fois l'ensemble ainsi mis en place, le générateur d'impulsions est raccordé à la sonde et au microcâble, avec l'une des bornes du circuit de détection/stimulation reliée à l'électrode distale de la sonde (électrode localisée dans le ventricule droit), et l'autre borne de ce même circuit reliée à la partie libre active du microcâble (partie localisée dans le ventricule gauche).

L'application entre ces deux électrodes d'impulsions de stimulation produira un champ électrique englobant une partie importante de la masse cardiaque, permettant donc une stimulation efficace du ventricule gauche. Dans une variante, la partie active du microcâble émergeant dans le ventricule gauche présente une longueur beaucoup plus faible (de l'ordre de 10 mm ou moins) avec une forme de boucle, de manière à plaquer l'extrémité du microcâble contre la paroi du septum (côté ventricule gauche), définissant ainsi un site de stimulation localisé. La stimulation reste endocavitaire, mais la mobilité du microcâble et la surface de ce dernier exposée à la circulation artérielle sont très fortement réduites.

Cette configuration de sonde peut être également appliquée au cas d'un défibrillateur. La partie libre active du microcâble est alors reliée à l'une des bornes du circuit de choc du générateur, dont l'autre borne est reliée : à un bobinage de type RV disposé dans le ventricule droit ; et/ou à un bobinage de type SVC disposé dans la veine cave supérieure au voisinage de l'oreillette droite ; et/ou au boîtier du générateur ; et/ou à l'électrode distale de la sonde si celle-ci présente une surface suffisante. Une telle configuration permet, avantageusement, d'englober une masse cardiaque maximale, en dépit de la surface d'électrode relativement réduite par rapport à une électrode de choc classique. En outre, le choc sera appliqué entre des électrodes situées de part et d'autre du septum, celui-ci étant pris "en tenaille" dans le champ électrique. Ceci permet d'augmenter encore l'efficacité de la défibrillation à énergie constante, ou bien de réduire notablement l'énergie du choc délivré et donc la douleur associée, par rapport à une configuration conventionnelle où le choc serait délivré entre le boîtier du générateur et les bobinages RV et/ou SVC.

Une autre application du microcâble décrit ci-dessus est exposée dans la demande française 10 59521 du 19 novembre 2010, au nom de la Demanderesse, pour une *"Sonde de stimulation d'une cavité gauche du coeur, implantable dans le réseau coronarien",* qui propose, pour stimuler la cavité gauche, d'introduire la sonde et le microcâble non plus directement dans la cavité à stimuler mais dans le réseau coronarien, la sonde étant prolongée par le microcâble dont la partie active et ses électrodes de stimulation est appliquée contre la paroi de l'épicarde, au niveau de la cavité gauche à stimuler. Le microcâble permet de s'affranchir des difficultés liées à la diminution progressive de diamètre du conduit au fur et à mesure de la progression de la sonde dans la veine coronaire sélectionnée, et de multiplier les points de contact avec l'épicarde, permettant ainsi de maximiser l'efficacité de la stimulation. On pallie ainsi les difficultés rencontrées avec les sondes conventionnelles pour trouver un site de stimulation satisfaisant, obtenir un bon contact électrique de l'électrode contre le tissu de l'épicarde, et maintenir ce contact malgré les variations ou sollicitations diverses au cours du temps.

L'invention concerne une configuration spécifique d'un ensemble de stimulation (sonde plus microcâble) particulièrement bien adapté à la mise en oeuvre de la technique que l'on vient d'exposer qui fait l'objet des demandes FR 10 53499 et 10 59521.

Le problème de l'invention consiste, dans un tel cas, à résoudre les difficultés suivantes :
- établissement de façon simple et fiable d'une connexion électrique entre, d'une part, le microcâble et l'électrode distale ou proximale de l'extrémité de la sonde et, d'autre part, les bornes d'un connecteur standardisé (par exemple de type IS-1, IS-4 ou DS-4) côté proximal de la sonde ;
- la possibilité de régler de façon précise la longueur émergeante du microcâble dans le ventricule gauche, par exemple dans une plage de longueurs allant de 5 mm (contact avec le septum côté ventricule gauche) à 120 mm (contact avec la paroi libre du ventricule gauche), ou dans le réseau coronaire ;
- garantie d'un guidage de poussée du microcâble lors de la ponction de la paroi du septum ou de la progression dans le réseau coronaire, en évitant tout risque de plicature de ce microcâble lorsqu'il n'est pas gainé par la sonde ;
- mode opératoire ne faisant appel qu'à des gestes simples et rapides, déjà connus des chirurgiens, et ne nécessitant pas de formation spécifique ou l'utilisation d'outils spéciaux ;
- possibilité de réintervention sur le microcâble pendant la pose de l'ensemble (possibilité de repositionnements multiples) ou de réintervention post-opératoire.

Aucun des dispositifs proposés jusqu'à présent dans l'état de la technique ne permet de répondre à ces diverses exigences.

Ainsi, les US 2001/0012958 A1 et US 2010/0069983 A1 mettent en oeuvre une sonde classique coulissante (corps de sonde formant contenu) dans une autre sonde classique (corps de sonde formant contenant) avec côté proximal un *side port* par lequel émerge le corps de sonde formant contenu.

Le US 2006/0064150 A1, de son côté, propose une solution avec deux sorties électriques unipolaires distinctes, avec une variante monoconnecteur obtenue en ajoutant un branche parallèle de type *bypass.*

Quant au US 2003/0023296 A1, il ne comporte pas de fonction télescopique, alors qu'il s'agit d'une composante essentielle du système envisagé ci-dessus.

Aucune de ces propositions permet d'établir de liaison électrique entre, d'une part, l'ensemble des électrodes distales et, d'autres part, un unique connecteur proximal de type IS1 ou IS4/DF4 standard évitant les inconvénients d'une connectique multi-branches et procurant un avantage majeur de simplicité de mise en oeuvre sur le champ opératoire (pas d'erreur de connexion à la tête du boitier, réduction du volume du système, etc.). En outre, ces documents n'abordent pas ou peu les deux questions critiques de l'étanchéité et de la continuité de la ligne électrique, qui sont par nature une source de complication d'un système télescopique. Au surplus, les solutions consistant à "empiler" des sondes classiques devant coulisser (donc avec un jeu de fonctionnement) conduisent à des tailles plus importantes, incompatibles avec les applications exposées plus haut. L'invention propose un ensemble de stimulation/défibrillation du ventricule gauche par voie endocavitaire ou depuis une veine du réseau coronarien, comprenant, de manière en elle-même connue, par exemple d'après le US 2006/0064150 A1 précité: un corps de sonde en matériau déformable, avec une lumière interne; côté distal, des moyens d'ancrage à une paroi d'une cavité cardiaque ou d'une veine du réseau coronarien ; côté proximal, un connecteur avec une première borne ; au moins un organe coulissant, logé et apte à coulisser dans la lumière du corps de sonde en s'étendant sur toute la longueur du corps de sonde et au-delà de l'extrémité distale de celui-ci, avec côté distal une partie libre active comprenant au moins une région dénudée ; et un insert formé sur le corps de sonde dans une région proximale de celui-ci.

L'insert comprend : côté proximal de l'intervalle, une première liaison électrique à ladite première borne du connecteur ; et des moyens de couplage, aptes à être déplacés sélectivement entre (i) une position desserrée, où l'organe coulissant est libre de coulisser dans la lumière du corps de sonde, et (ii) une position serrée, où l'organe coulissant est à la fois mécaniquement immobilisé dans le corps de sonde et électriquement connecté à ladite première liaison électrique.

De façon caractéristique de l'invention : l'organe coulissant est un microcâble électriquement conducteur ; le corps de sonde comprend au moins un conducteur isolé s'étendant sur toute la longueur de corps de sonde, ainsi qu'au moins une électrode de stimulation/défibrillation ; le connecteur comprend une seconde borne reliée à ladite électrode de stimulation/défibrillation via ledit conducteur isolé s'étendant le long du corps de sonde. En outre, l'insert est électriquement isolé et comprend : un intervalle d'interruption du conducteur isolé ; côté proximal de l'intervalle, une seconde liaison électrique à ladite seconde borne du connecteur ; côté distal de l'intervalle, une troisième liaison électrique au conducteur isolé ; et une connexion, en contournement de l'intervalle, de la seconde liaison électrique à la troisième liaison électrique.

Selon un certain nombre de caractéristiques subsidiaires avantageuses:
- les moyens de couplage comprennent une vis orientée radialement par rapport à la direction du corps de sonde, avec une extrémité interne apte à pénétrer dans l'intervalle pour venir en appui radial contre le microcâble, et un extrémité externe comprenant une empreinte apparente apte à recevoir un outil de vissage ;
- l'ensemble comprend en outre un mandrin de poussée du microcâble dans le corps de sonde, et le microcâble est terminé à son extrémité proximale par un réceptacle apte à recevoir l'extrémité distale du mandrin de poussée, par exemple un réceptacle en forme de cône ressort creux, coopérant de manière vissante avec l'extrémité distale du mandrin de poussée ; en variante, le mandrin est terminé à son extrémité distale par une douille créée par déformation locale d'un hypotube, et apte à être insérée directement, en force contrôlée, sur le microcâble.
- le corps de sonde et le microcâble sont dimensionnés de manière que, lorsque côté distal l'extrémité du microcâble affleure le débouché de la lumière du corps de sonde, côté proximal le microcâble se trouve alors entièrement introduit dans l'ensemble formé par le corps de sonde et le connecteur ;
- le microcâble comprend au voisinage de son extrémité proximale, dans la région comprise entre cette extrémité proximale et l'insert, une bague de butée solidaire du microcâble et de diamètre supérieur au diamètre de l'alésage de l'insert recevant le microcâble côté proximal, de manière à limiter le coulissement du microcâble dans le corps de sonde, et par voie de conséquence la longueur de la partie émergeant au-delà de l'extrémité distale de celui-ci, à une longueur maximale prédéterminée ;

Dans une variante de réalisation, la sonde est réalisée en deux parties séparables, avec (i) une partie formant sonde proprement dite terminée côté proximal par une première fiche, et (ii) une partie formant prolongateur avec côté distal une seconde fiche homologue de la première fiche et côté proximal ledit connecteur, et dans laquelle l'insert est formé sur la partie formant prolongateur.

On va maintenant décrire un exemple de mise en oeuvre du dispositif de l'invention, en référence aux dessins annexés où les mêmes références numériques désignent d'une figure à l'autre des éléments identiques.
La Figure 1 est une vue schématique globale de l'ensemble selon l'invention, montrant, depuis l'extrémité distale jusqu'à l'extrémité proximale, les différents éléments composant cet ensemble.
La Figure 2 illustre, sous forme agrandie et partiellement arrachée, l'extrémité distale de l'ensemble de la Figure 1.
La Figure 3 illustre, sous forme agrandie et partiellement arrachée, la partie proximale située avant le connecteur de l'ensemble de la Figure 1, montrant notamment la manière dont l'extrémité du microcâble peut être assujettie à un mandrin de poussée.
La Figure 4 est une vue agrandie de l'insert de la Figure 3, interposé sur le corps de sonde pour permettre l'immobilisation et la prise de contact contrôlée du microcâble dans le corps de sonde.

On va maintenant décrire un exemple de réalisation de l'ensemble selon l'invention, destiné à la stimulation/défibrillation endocavitaire du ventricule gauche.

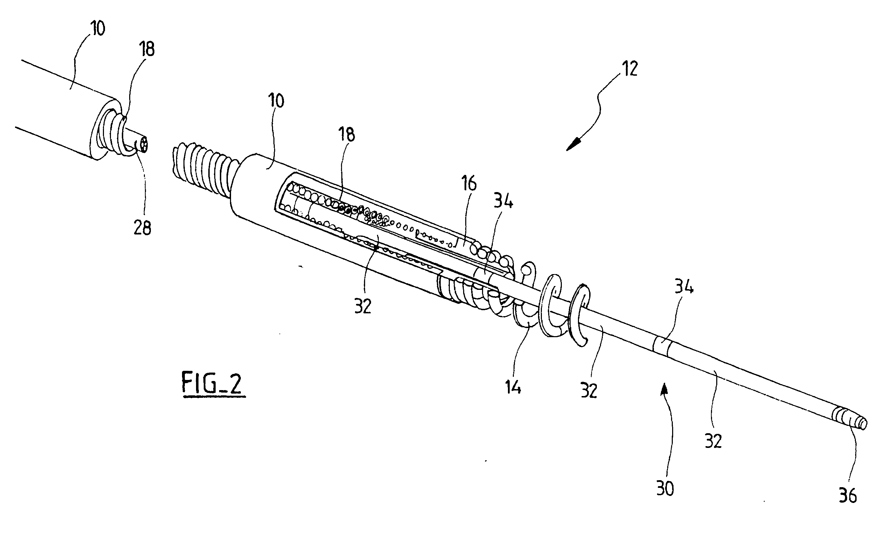

Cet ensemble comporte tout d'abord une sonde dont la partie distale au moins est de structure conventionnelle, correspondant par exemple aux modèles *Stelid BS46D ou Beflex RF46D* commercialisés par Sorin CRM, Clamart, France (ces modèles n'étant toutefois pas à conducteurs isolés). Cette sonde comporte un corps de sonde 10 de structure conventionnelle, avec une gaine 10 en matériau déformable, généralement une gaine en polyuréthanne ou silicone. Le corps de sonde est terminé à son extrémité distale 12 (représentée plus en détail Figure 2) par une tête de sonde comprenant une vis hélicoïdale 14 à spires non jointives dont le diamètre est par exemple de l'ordre de 1 à 2 mm. Cette vis est solidarisée à la tête de sonde par un embout 16 comportant en son centre une lumière et des moyens d'étanchéité, par exemple un bouchon percé en silicone, permettant d'éviter tout reflux de sang à l'intérieur du corps de sonde aussi bien en l'absence qu'en présence d'un élément introduit dans la lumière centrale (comme cela est illustré Figure 2).

L'embout métallique 16 fait également office d'électrode distale, et cette électrode (ou bien une électrode annulaire spécifique portée par la tête de sonde) est électriquement reliée à un conducteur interne isolé 18, par exemple un conducteur spiralé s'étendant sur toute la longueur de la gaine jusqu'à l'extrémité proximale 20, opposée, de la sonde (ce conducteur est par exemple du même type que celui du modèle de sonde *Xfine TX26D* commercialisé par Sorin CRM, Clamart, France). Cette extrémité 20 est terminée par un connecteur électrique 22 destiné à être couplé au boîtier d'un générateur implanté tel qu'un stimulateur/défibrillateur ou re-synchroniseur.

Le matériau et les dimensions de la gaine du corps de sonde 10 peuvent être choisis, en combinaison avec le conducteur spiralé 18, pour procurer une certaine rigidité en torsion du corps de sonde, de manière à pouvoir transmettre un couple de torsion depuis l'extrémité proximale 20 de la sonde (au niveau du connecteur électrique 22) jusqu'à l'extrémité distale 12 de la tête de sonde. Cette rotation doit permettre d'entraîner en rotation la vis 14 pour la faire pénétrer par vissage dans le tissu cardiaque. Le connecteur 22 comporte deux électrodes (au moins), avec une électrode annulaire 24 ou électrode *"ring"* destinée à être reliée à l'électrode 16 portée par la tête de sonde, et une électrode d'extrémité 26 ou électrode "*tip*" destinée à être reliée au microcâble qui sera introduit dans le corps de sonde. On décrira plus bas la manière, caractéristique de l'invention, dont ces deux liaisons électriques respectives sont établies. Comme illustré sur les figures, un microcâble 28 est introduit dans la lumière centrale du corps de sonde 10, jusqu'à l'embout 16 et au travers du bouchon en silicone percé porté par ce dernier, de manière à émerger au-delà de l'extrémité distale du corps de sonde sur une partie libre 30 dont on pourra contrôler l'émergence (initialement, le microcâble vient en butée contre la paroi du septum, et il est orienté sensiblement dans la direction de l'axe central de la vis 14).

Le diamètre typique du coeur du microcâble 28, c'est-à-dire de la partie métallique non isolée de celui-ci, est de l'ordre de 0,1 à 0,3 mm. L'isolation de ce coeur est assurée par une gaine isolante de type parylène, polyuréthanne, silicone ou ETFE, conduisant à un diamètre total de l'ordre de 0,3 à 0,5 mm pour le microcâble avec sa gaine.

Ces dimensions sont à comparer au diamètre de l'ordre de 0,8 mm des aiguilles de ponction des kits transseptaux standards (perçage du septum auriculaire avec accès par la voie fémorale), et à celui des cathéters 7 French (2,33 mm) ou 9 French (3 mm) et des sondes 5 French (1,66 mm) ou 6 French (2 mm) utilisés dans les techniques classiques de pose d'une sonde endocavitaire gauche avec approche transseptale. Le microcâble de l'invention, même revêtu d'une gaine isolante, est donc d'un diamètre très inférieur à ce qui est usuellement utilisé pour les interventions transseptales.

Le microcâble est avantageusement réalisé à base de nitinol (alliage Ni-Ti), matériau dont l'avantage essentiel est son extrême endurance en fatigue. Plus précisément, la structure du microcâble est une structure multifilaire dans laquelle chaque brin est constitué d'une âme en platine-iridium (pour la radio-opacité) gainée par une épaisseur de nitinol (ou inversement), l'ensemble pouvant alors être recouvert soit par une fine couche de parylène (par exemple de type C), soit par un tube polyuréthanne.

Ces types de microcâbles sont disponibles par exemple auprès de la société Fort Wayne Metals Inc., Fort Wayne, USA, et sont utilisés dans le domaine médical notamment pour réaliser des conducteurs de défibrillation - toutefois selon un arrangement de matériaux différent : dans ces applications connues, la structure est une structure multifilaire dans laquelle chaque brin inclut une âme en argent (pour améliorer la conductivité) gainée par une épaisseur d'inox ; ces microstructures, isolées ou non, sont ensuite incorporées dans un corps de sonde multi-lumière de construction classique.

Les avantages de la structure de microcâble décrite plus haut résident dans le fait que les éléments les moins endurants mécaniquement (platine iridié ou argent) sont encapsulés directement dans la gaine en nitinol. Les conséquences d'une fracture éventuelle de ces brins sont donc minimisées.

Comme alternative, il est néanmoins possible de disposer un brin en platine-iridium au centre d'une structure multifilaire de type 1x7, le brin le plus fragile étant alors enlacé par les brins externes plus endurants.

Enfin, le platine-iridium peut être remplacé par tout matériau radio-opaque tel que le tantale, et le nitinol peut être remplacé par des matériaux moins performants en endurance mais néanmoins suffisants ou moins chers tels que l'acier inox MP35N, couramment utilisé dans la fabrication des conducteurs standard.

La partie active 30 du microcâble 28 (sa partie émergeante) comporte une pluralité de parties dénudées formant une succession d'électrodes individuelles électriquement reliées ensemble. Par exemple, dans l'exemple (non limitatif) représenté sur les figures, le revêtement isolant 32 est interrompu de manière à définir une succession de courtes zones annulaires dénudées 34, le microcâble étant terminé par l'électrode distale 36.

Cette configuration peut être réalisée en enfilant successivement et alternativement des tubes isolants (pour former les régions 32) et des bagues conductrices, par exemple serties sur le microcâble (pour former les régions dénudées 34). En variante, notamment dans le cas où le coeur du microcâble est revêtu par une fine couche isolante de parylène, la configuration est réalisée en ménageant des ouvertures dans l'isolant le long du microcâble, par exemple par ablation plasma, pour former les zones dénudées 34 et 36. Pour améliorer les performances électriques, ces zones peuvent être en outre revêtues par exemple de nitrure de titane.

La configuration que l'on vient de décrire correspond, pour l'essentiel à celle décrite dans la demande FR 10 53499 précitée.

On va maintenant décrire les aspects caractéristiques de la présente invention.

Il s'agit de mettre à disposition du chirurgien un moyen simple et fiable d'établir une connexion électrique entre le microcâble et la borne correspondante du connecteur 22 après avoir réglé précisément la longueur émergente de la partie active du microcâble à la valeur choisie.

Pour cela, l'invention propose d'interposer sur le corps de sonde 10 un insert, réalisé monobloc avec le corps de sonde.

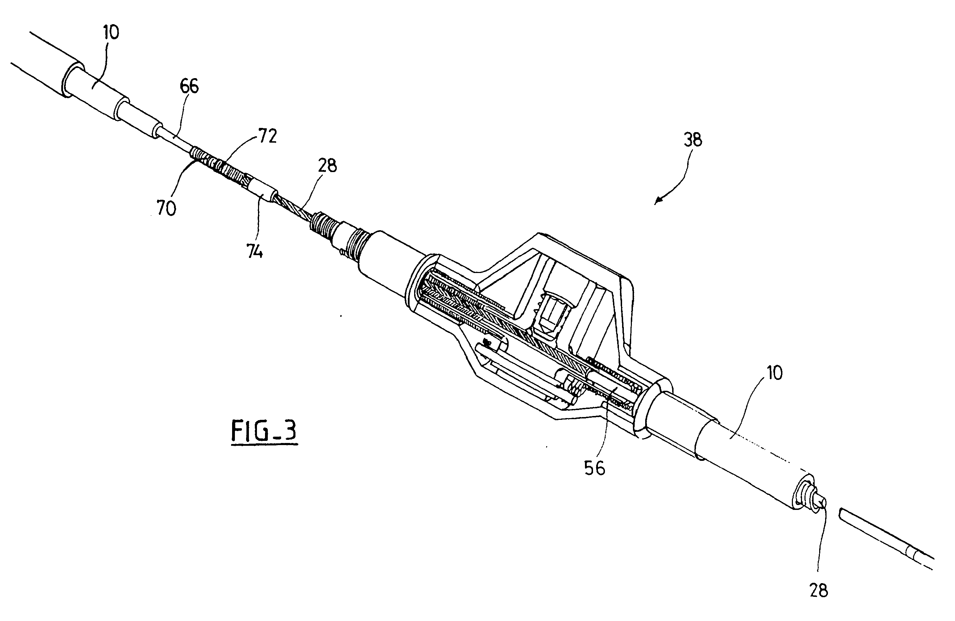

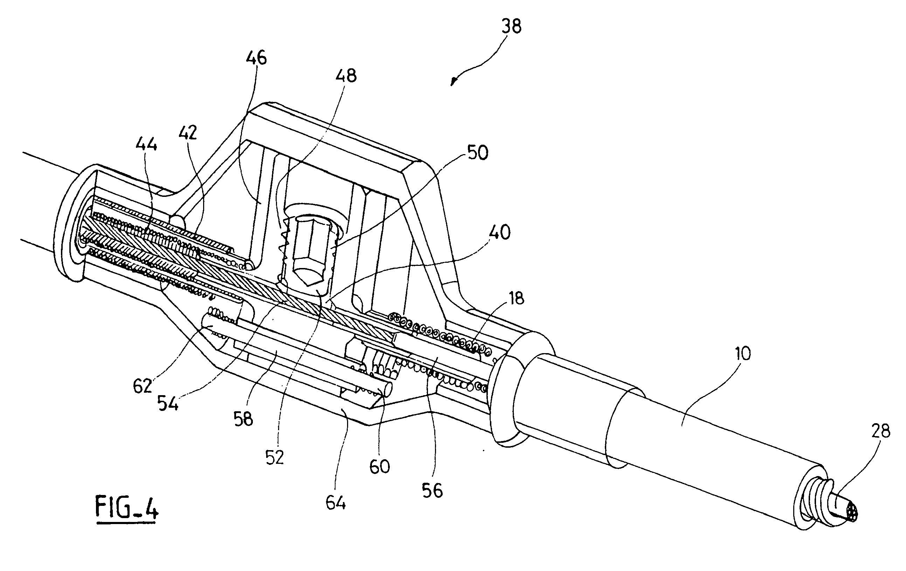

Cet insert 38, représenté en détail notamment Figures 3 et 4, est situé dans la région proximale du corps de sonde, au voisinage du connecteur 20, par exemple à environ 10 cm de l'extrémité distale (c'est-à-dire de l'extrémité terminale de la borne 26) du connecteur, dans la configuration illustrée.

L'insert 38 comprend, comme on peut le voir en particulier sur la vue arrachée de la Figure 4, un espace intérieur définissant un intervalle 40 d'interruption du conducteur spiralé 18 - conducteur qui, dans une sonde conventionnelle, relie directement l'électrode située côté distal de la sonde au connecteur situé côté proximal de cette même sonde.

D'un premier côté (côté proximal) de l'intervalle 40, on trouve une première liaison électrique 42 constituée d'un conducteur spiralé relié à une borne correspondante du connecteur 22, à savoir la borne annulaire *ring* 24 ; de ce même côté, on trouve également une deuxième liaison 44, par exemple en forme de connecteur spiralé, à l'autre borne du connecteur 22, à savoir la borne d'extrémité *tip* 26. Les deux liaisons 42 et 44 sont des liaisons coaxiales.

De l'autre côté (côté distal) de l'intervalle 40, on trouve une troisième liaison électrique, à savoir le conducteur isolé spiralé 18 électriquement relié à l'électrode portée par l'extrémité distale du corps de sonde (l'électrode constituée par l'embout métallique 16 portant la vis 14, dans l'exemple illustré Figure 2).

L'insert 38 comprend également un corps 46 en matériau conducteur auquel est électriquement reliée la seconde liaison électrique 44. Ce corps 46 comporte un orifice radial taraudé 48 recevant une vis 50 s'étendant perpendiculairement au corps de sonde 10 et donc au microcâble 28. Cette vis présente côté intérieur une extrémité aplatie 52 apte à venir en contact mécanique et électrique avec une région non isolée 52 du microcâble introduit à l'intérieur du corps de sonde 10 et de l'insert 38. Du côté opposé, la vis 50 comporte une empreinte permettant sa manoeuvre par un outil conventionnel de type tournevis débrayable à limitation de couple. En dehors de la région 54, le microcâble est isolé par le revêtement 56. L'insert 38 comporte également une connexion 58, décalée axialement par rapport à l'axe principal de l'insert et du corps de sonde (donc par rapport au microcâble). Cette connexion 58 permet de contourner l'intervalle 40 et elle est pour cela reliée côté distal en 60 au conducteur spiralé isolé 18 localement dénudé, et côté proximal en 62 à la seconde liaison électrique 42.

Par ailleurs, les différents organes de l'insert 38 sont enfermés dans une enveloppe étanche 64, par exemple en silicone surmoulé avec le corps de sonde 10, dotée d'une fente permettant le passage du tournevis débrayable.

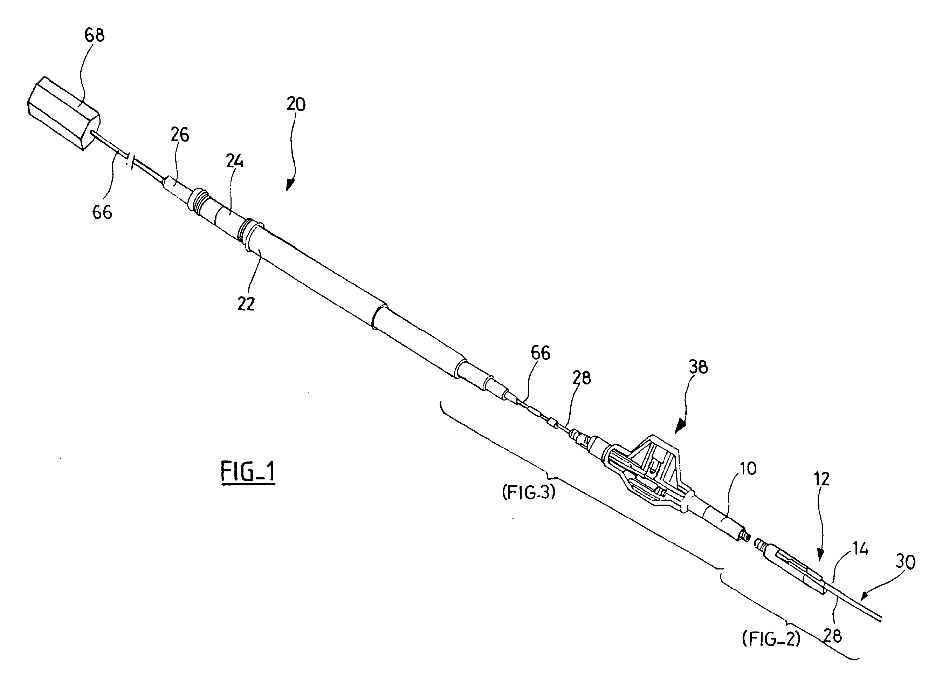

Pour sa manipulation, le microcâble est connecté à un mandrin de poussée 66 (Figures 1 et 3), qui est un mandrin court terminé côté proximal par une poignée de manoeuvre 68 et côté distal par un embout 70 spiralé. Comme illustré Figure 3, ce mandrin de poussée 66 peut être couplé à l'extrémité proximale du microcâble 28 grâce à un cône-ressort 72 formé à cette extrémité du microcâble, et dans lequel peut être vissée l'extrémité spiralée 70 du mandrin 66, de manière à établir ainsi une liaison temporaire entre le microcâble et le mandrin de poussée. En variante, le mandrin est terminé à son extrémité distale par une douille créée par déformation locale d'un hypotube, cette douille étant alors insérée directement, en force contrôlée, sur le microcâble.

On notera que l'extrémité atraumatique formée par la spirale de vissage 70 à l'extrémité du mandrin de poussée 66 protège la lumière interne du corps de sonde de tout risque de perforation. Par ailleurs, la partie proximale du corps du mandrin de poussée peut être légèrement courbée, afin de produire une légère friction d'immobilisation avec la lumière interne du corps de sonde, permettant d'éviter la chute du microcâble hors du champ stérile d'intervention.

L'ensemble sonde-microcâble-mandrin de poussée est dimensionné de telle sorte que lorsque l'extrémité distale du microcâble affleure l'extrémité du corps de sonde et vient en contact avec le septum, le mandrin de poussée se trouve déjà introduit dans le connecteur sur une longueur de 10 mm par exemple. Cette configuration permet d'effectuer une poussée efficace de ponction mécanique du septum grâce au mandrin de poussée rigide, le microcâble (souple) étant alors complètement gainé par le corps de sonde, donc apte à transmettre l'effort de compression tout en étant protégé des risques de plicature (formation de plis) pouvant altérer son endurance.

Par ailleurs, le cône-ressort 72 du microcâble est prolongé par une bague 74 par exemple en platine, sertie sur le microcâble 28, et dont le diamètre est supérieur à l'alésage axial de l'insert 38. Cette configuration garantit que le microcâble n'émergera pas dans le ventricule gauche au-delà d'une longueur maximale, et permet également d'identifier sous rayons X son extrémité proximale.

Le revêtement isolant 56 sur le microcâble 28 commence à environ 10 cm de l'extrémité proximale de celui-ci (cône de vissage 72), de manière à garantir une connexion électrique après serrage de la vis quelle que soit la longueur émergeante (longueur elle-même limitée par la venue en butée de la bague 74). En d'autres termes, quelle que soit cette longueur émergeante, la partie du microcâble 28 qui se trouvera en vis-à-vis de la face d'appui 52 de la vis 50 sera toujours une partie dénudée 54, permettant ainsi d'assurer un contact électrique entre le microcâble et la vis 50 et, par voie de conséquence, avec la seconde liaison 44.

La configuration que l'on vient de décrire assure donc une liaison mécanique et électrique apte à appliquer si nécessaire l'énergie RF de ponction du septum, et en outre à tester les performances électriques au moment de la pose de la sonde (*mapping*), tout en permettant un réglage de la longueur émergeante du microcâble dans une plage comprise entre quelques millimètres (configuration de stimulation du septum interventriculaire par le côté du ventricule gauche) et environ 120 mm (stimulation de la paroi libre du ventricule gauche).

On va maintenant décrire le mode opératoire pour la mise en oeuvre de cet ensemble.

La première phase est une phase classique de pose de la sonde, qui consiste tout d'abord à repérer le site d'ancrage, en manipulant l'extrémité distale 12 de la sonde par l'intermédiaire d'un mandrin classique introduit dans la lumière centrale de la sonde. L'ensemble est introduit dans la veine cave supérieure, l'oreillette droite puis le ventricule jusqu'à venir en butée contre la paroi du septum interventriculaire.

Une fois ce site atteint, le praticien imprime une rotation axiale au corps de sonde, ce qui va avoir pour effet de faire pénétrer la vis hélicoïdale 14 dans la paroi du septum, le vissage complet étant détecté tactilement par le praticien du fait de la résistance opposée à la rotation. Le mouvement de rotation axiale est imprimé, selon le cas, (i) soit directement au corps de sonde 10 (gaine et conducteur spiralé 18), (ii) soit à la fiche du connecteur, pour une sonde de type *pin driven* dans le cas où côté proximal la fiche de connexion est solidaire d'un conducteur axial s'étendant à l'intérieur du corps de sonde, ce conducteur étant lui-même libre en rotation et relié à la vis hélicoïdale à son extrémité distale.

Le site est ensuite confirmé par examen radiographique selon différentes inclinaisons ; si la position n'est pas satisfaisante le praticien peut dévisser la tête de sonde et déplacer celle-ci sous contrôle vers un autre point, puis tester le nouveau site.

Le mandrin est ensuite retiré, et remplacé par le microcâble qui est introduit dans la lumière centrale de la sonde jusqu'à venir en contact avec le septum.

Après avoir orienté le microcâble au contact de la paroi du septum, ce microcâble est relié côté proximal à un générateur de ponction RF. L'énergie RF produite par le générateur est appliquée à l'extrémité distale du microcâble, et cette énergie va permettre la réalisation d'une ponction très fine dans le septum interventriculaire, le diamètre de cette ponction étant défini par le diamètre du microcâble, de l'ordre de 0,3 à 0,5 mm comme on l'a indiqué plus haut.

Après que la paroi du septum ait été complètement traversée, le générateur de ponction RF est arrêté et déconnecté du microcâble.

L'étape suivante consiste à pousser le microcâble au-delà du septum, dorénavant traversé de part en part, de manière à laisser émerger la partie libre 30 dans le volume intérieur du ventricule gauche au-delà de la partie intermédiaire enserrée dans le septum, et ceci sur une longueur contrôlée, typiquement comprise entre quelques millimètres et une dizaine de centimètres, selon la configuration de stimulation choisie (stimulation du ventricule gauche par sa paroi libre, ou bien par sa paroi septale).

Une fois le microcâble positionné à l'emplacement voulu, le chirurgien serre la vis 50 de l'insert 38. Le microcâble 28 est alors immobilisé et solidarisé en rotation (et également en translation) par rapport au corps de sonde 10, ce qui permet un dévissage manuel aisé du mandrin de poussée 66. L'effort de compression exercé par la vis de serrage 50, et donc la contrainte exercée sur le microcâble, est contrôlé par le couple de débrayage du tournevis dynamométrique utilisé.

Du point de vue mécanique, on notera que la fonction de la sonde est multiple :
- sélection du site de ponction du septum;
- guidage du microcâble lors du trajet intraveineux ;
- transmission de la poussée jusqu'à l'extrémité du microcâble pendant la ponction ;
- stabilisation du microcâble pendant la ponction RF du septum;
- définition (orientation) de la trajectoire du microcâble pendant la ponction RF, avec possibilité de tester plusieurs configurations d'implantation ;
- enfin, stabilisation et protection de la grande longueur du microcâble côté veineux pendant toute la durée de vie du dispositif.

Il est également à noter que le système est parfaitement réversible, aussi bien pendant l'intervention qu'en post-opératoire.

Du point de vue électrique, une fois serrée la vis 50 de l'insert 38, on se trouve en présence d'une configuration à deux lignes, avec :
- une ligne de stimulation comprenant, successivement : le microcâble 28 (dont la partie active émerge dans le ventricule gauche), la vis 50, la seconde liaison 44, et la borne d'extrémité *tip* 26 du connecteur 22 ; et
- une ligne bipolaire comprenant, successivement : l'électrode bipolaire portée par la tête de sonde et constituée par l'embout 16 portant la vis: 14 (en contact avec la paroi du ventricule droit), le conducteur spiralé isolé 18, la connexion 58 en contournement de l'intervalle d'interruption 40, la première liaison 42, et la borne annulaire *ring* 24 du connecteur 22.

L'isolement des deux lignes au niveau de l'insert 38 est obtenu de façon classique par collage silicone.

L'ensemble que l'on vient de décrire peut, en variante, être implémenté sur un prolongateur de sonde au lieu de l'être directement sur la sonde elle-même.

Dans ce cas, l'insert 38 est disposé sur un prolongateur de type IS-1 bipolaire/IS-1 bipolaire qui est connecté à une sonde conventionnelle, ce qui permet l'utilisation d'un microcâble avec une sonde ventriculaire gauche déjà implantée.

Dans une application à une sonde de stimulation implantable dans le réseau coronarien, telle que celle exposée dans la demande française 10 5952 précitée, la vis d'ancrage à l'extrémité distale de la sonde est remplacée par un moyen de retenue dans la veine de cette extrémité. Ce moyen de retenue est par exemple constitué d'une vis en silicone du même type que celui utilisé par la sonde *Situs LV* commercialisée par Sorin CRM (Clamart, France) et décrit dans le EP 0 993 840 A1 (ELA Midical). D'autre part, cette extrémité ne comporte pas d'électrode, et les divers éléments correspondants décrits ci-dessus pourront être omis. Enfin, qu'il s'agisse d'une de stimulation par voie endocavitaire ou depuis une veine du réseau coronarien, l'invention est applicable, *mutatis mutandis,* à une sonde pourvue d'une pluralité de microcâbles disposés dans une pluralité correspondante de lumière internes du corps de sonde, en prévoyant pour les divers microcâbles un nombre correspondant de liaisons électriques et d'inserts respectifs.

## Revendications

1. Un ensemble de stimulation/défibrillation du ventricule gauche par voie endocavitaire ou depuis une veine du réseau coronarien, comprenant :
- un corps de sonde (10) en matériau déformable, avec une lumière interne;
- le corps de sonde comprenant au moins un conducteur isolé (18) s'étendant sur toute la longueur de corps de sonde, ainsi qu'au moins une électrode (16) de stimulation/défibrillation ;
- côté distal (12), des moyens d'ancrage (14) à une paroi d'une cavité cardiaque ou d'une veine du réseau coronarien ;
- côté proximal, un connecteur (22) avec une première borne (26) ;
- au moins un organe coulissant (28), logé et apte à coulisser dans la lumière du corps de sonde en s'étendant sur toute la longueur du corps de sonde et au-delà de l'extrémité distale de celui-ci, avec côté distal une partie libre active (30) comprenant au moins une région dénudée (34, 36) ; et
- un insert (38) formé sur le corps de sonde dans une région proximale de celui-ci, comprenant :
• côté proximal de l'insert, une première liaison électrique (44) à ladite première borne (26) du connecteur ; et
• des moyens de couplage (48, 50, 52), aptes à être déplacés sélectivement entre (i) une position desserrée, où l'organe coulissant est libre de coulisser dans la lumière du corps de sonde, et (ii) une position serrée, où l'organe coulissant est à la fois mécaniquement immobilisé dans le corps de sonde et électriquement connecté à ladite première liaison électrique (44),
ensemble **caractérisé en ce que** :
- l'organe coulissant est un microcâble (28) électriquement conducteur ;
- le connecteur (22) comprend une seconde borne (24) reliée à ladite électrode de stimulation/défibrillation via ledit conducteur isolé s'étendant le long du corps de sonde ; et
- l'insert est électriquement isolé et comprend :
• un intervalle (40) d'interruption du conducteur isolé ;
• côté proximal de l'intervalle, une seconde liaison électrique (42) à ladite seconde borne (24) du connecteur ;
• côté distal de l'intervalle, une troisième liaison électrique (18) au conducteur isolé ; et
• une connexion (58), en contournement de l'intervalle, de la seconde liaison électrique à la troisième liaison électrique.

2. L'ensemble de la revendication 1, dans lequel les moyens de couplage comprennent une vis (48) orientée radialement par rapport à la direction du corps de sonde, avec une extrémité interne (52) apte à pénétrer dans l'intervalle pour venir en appui radial contre le microcâble, et une extrémité externe comprenant une empreinte apparente apte à recevoir un outil de vissage.

3. L'ensemble de la revendication 1, dans lequel :
- il comprend en outre un mandrin (66) de poussée du microcâble dans le corps de sonde, et
- le microcâble est terminé à son extrémité proximale par un réceptacle (72) apte à recevoir l'extrémité distale (70) du mandrin de poussée, ou par une douille créée par déformation locale d'un hypotube et insérée en force contrôlée directement sur le microcâble.

4. L'ensemble de la revendication 3, dans lequel le réceptacle est en forme de cône ressort creux (72), coopérant de manière vissante avec l'extrémité distale du mandrin de poussée.

5. L'ensemble de la revendication 1, dans lequel :
- il comprend en outre un mandrin de poussée du microcâble dans le corps de sonde, et
- le mandrin est terminé à son extrémité distale par une douille créée par déformation locale d'un hypotube, et apte à être insérée directement, en force contrôlée, sur le microcâble.

6. L'ensemble de la revendication 1, dans lequel le corps de sonde et le microcâble sont dimensionnés de manière que, lorsque côté distal l'extrémité du microcâble affleure le débouché de la lumière du corps de sonde, côté proximal le microcâble se trouve alors entièrement introduit dans l'ensemble formé par le corps de sonde et le connecteur.

7. L'ensemble de la revendication 1, dans lequel le microcâble comprend au voisinage de son extrémité proximale, dans la région comprise entre cette extrémité proximale et l'insert, une bague de butée (74) solidaire du microcâble et de diamètre supérieur au diamètre de l'alésage de l'insert recevant le microcâble côté proximal, de manière à limiter le coulissement du microcâble dans le corps de sonde, et par voie de conséquence la longueur de la partie émergeant au-delà de l'extrémité distale de celui-ci, à une longueur maximale prédéterminée.

8. L'ensemble de la revendication 1, dans lequel la sonde est réalisée en deux parties séparables, avec (i) une partie formant sonde proprement dite terminée côté proximal par une première fiche, et (ii) une partie formant prolongateur avec côté distal une seconde fiche homologue de la première fiche et côté proximal ledit connecteur, et dans laquelle l'insert est formé sur la partie formant prolongateur.

## Claims

1. Assembly for stimulating/defibrillating the left ventricle by endocavity pathway or from a vein of the coronary network, comprising:
- a probe body (10) made of deformable material, with an internal opening;
- the probe body comprising at least one insulated conductor (18) extending over the entire length of the probe body, as well as at least one stimulation/defibrillation electrode (16);
- on the distal side (12), means (14) for anchoring to a wall of a cardiac cavity or of a vein of the coronary network;
- on the proximal side, a connector (22) with a first terminal (26);
- at least one sliding member (28), housed and suitable for sliding in the opening of the probe body by extending over the entire length of the probe body and beyond the distal end thereof, with, at the distal end, an active free part (30) comprising at least one stripped region (34, 36); and
- an insert (38) formed on the probe body in a proximal region thereof, comprising:
• on the proximal side of the insert, a first electrical link (44) to said first terminal (26) of the connector; and
• coupling means (48, 50, 52), capable of being moved selectively between (i) a loose position, where the sliding member is free to slide in the opening of the probe body, and (ii) a tight position, where the sliding member is both mechanically immobilized in the probe body and electrically connected to said first electrical link (44),
the assembly being **characterized in that**:
- the sliding member is an electrically conductive microcable (28);
- the connector (22) comprises a second terminal (24) linked to said stimulation/defibrillation electrode via said insulated conductor extending along the probe body; and
- the insert is electrically insulated and comprises:
• an interval (40) interrupting the insulated conductor;
• on the proximal side of the interval, a second electrical link (42) to said second terminal (24) of the connector;
• on the distal side of the interval, a third electrical link (18) to the insulated conductor; and
• a connection (58), circumventing the interval, from the second electrical link to the third electrical link.

2. Assembly of Claim 1, in which the coupling means comprise a screw (48) oriented radially in relation to the direction of the probe body, with an internal end (52) suitable for penetrating into the interval to bear radially against the microcable, and an external end comprising a visible imprint suitable for receiving a screwing tool.

3. Assembly of Claim 1, in which:
- it also comprises a mandrel (66) for thrusting the microcable into the probe body, and
- the microcable is terminated at its proximal end by a receptacle (72) suitable for receiving the distal end (70) of the thrust mandrel, or by a bush created by local deformation of a hypotube and inserted with controlled force directly onto the microcable.

4. Assembly of Claim 3, in which the receptacle is in the form of a hollow spring cone (72), cooperating in a screwing manner with the distal end of the thrust mandrel.

5. Assembly of Claim 1, in which:
- it also comprises a mandrel for thrusting the microcable into the probe body, and
- the mandrel is terminated at its distal end by a bush created by local deformation of a hypotube, and suitable for being inserted directly, with controlled force, onto the microcable.

6. Assembly of Claim 1, in which the probe body and the microcable are dimensioned in such a way that, when, on the distal side, the end of the microcable is flush with the end of the opening of the probe body, and on the proximal side, the microcable is then entirely introduced into the assembly formed by the probe body and the connector.

7. Assembly of Claim 1, in which the microcable comprises, in the vicinity of its proximal end, in the region included between this proximal end and the insert, an abutment ring (74) attached to the microcable and of a diameter greater than the diameter of the bore of the insert receiving the microcable on the proximal side, so as to limit the sliding of the microcable in the probe body, and consequently the length of the part that emerges beyond the distal end thereof, to a predetermined maximum length.

8. Assembly of Claim 1, in which the probe is produced in two separable parts, with (i) an actual probe-forming part terminated on the proximal side by a first plug, and (ii) an extender-forming part with, on the distal side, a second plug similar to the first plug and, on the proximal side, said connector, and in which the insert is formed on the extender-forming part.

## Patentansprüche

1. Einheit zur endokavitären oder von einer Vene des Koronarvenennetzes ausgehenden Stimulation/Defibrillation des linken Ventrikels, die enthält:
- einen Sondenkörper (10) aus verformbarem Material mit einer inneren Öffnung;
- wobei der Sondenkörper mindestens einen isolierten Leiter (18), der sich über die ganze Länge des Sondenkörpers erstreckt, sowie mindestens eine Stimulations-/Defibrillationselektrode (16) enthält;
- auf der distalen Seite (12) Befestigungseinrichtungen (14) an einer Wand einer Herzkammer oder einer Vene des Koronarvenennetzes;
- auf der proximalen Seite einen Verbinder (22) mit einem ersten Anschluss (26);
- mindestens ein gleitendes Organ (28), das in der Öffnung des Sondenkörpers angeordnet ist und darin gleiten kann, indem es sich über die ganze Länge des Sondenkörpers und über dessen distales Ende hinaus erstreckt, mit auf der distalen Seite einem aktiven freien Bereich (30), der mindestens eine abisolierte Zone (34, 36) enthält; und
- einen auf dem Sondenkörper in einer proximalen Zone von diesem geformten Einsatz (38), der enthält:
auf der proximalen Seite des Einsatzes eine erste elektrische Verbindung (44) mit dem ersten Anschluss (26) des Verbinders; und
. Koppeleinrichtungen (48, 50, 52), die selektiv zwischen (i) einer gelösten Stellung, in der das gleitende Organ frei in der Öffnung des Sondenkörpers gleiten kann, und (ii) und einer eingespannten Stellung verschoben werden können, in der das gleitende Organ sowohl mechanisch im Sondenkörper blockiert als auch elektrisch mit der ersten elektrischen Verbindung (44) verbunden ist,
wobei die Einheit **dadurch gekennzeichnet ist, dass**:
- das gleitende Organ ein elektrisch leitendes Mikrokabel (28) ist;
- der Verbinder (22) einen zweiten Anschluss (24) enthält, der mit der Stimulations-/Defibrillationselektrode über den sich entlang des Sondenkörpers erstreckenden isolierten Leiter verbunden ist; und
- der Einsatz elektrisch isoliert ist und enthält:
. einen Unterbrechungszwischenraum (40) des isolierten Leiters;
. auf der proximalen Seite des Zwischenraums eine zweite elektrische Verbindung (42) mit dem zweiten Anschluss (24) des Verbinders;
. auf der distalen Seite des Zwischenraums eine dritte elektrische Verbindung (18) mit dem isolierten Leiter; und
. eine Verbindung (58), mit Umgehung des Zwischenraums, der zweiten elektrischen Verbindung mit der dritten elektrischen Verbindung.

2. Einheit nach Anspruch 1, wobei die Koppeleinrichtungen eine Schraube (48) enthalten, die bezüglich der Richtung des Sondenkörpers radial ausgerichtet ist, mit einem inneren Ende (52), das in den Zwischenraum eindringen kann, um in radiale Auflage gegen das Mikrokabel zu kommen, und mit einem äußeren Ende, das eine sichtbare Mulde enthält, die ein Schraubwerkzeug aufnehmen kann.

3. Einheit nach Anspruch 1, wobei:
- sie außerdem einen Dorn (66) zum Schieben des Mikrokabels in den Sondenkörper enthält, und
- das Mikrokabel an seinem proximalen Ende in einem Aufnahmebehälter (72), der das distale Ende (70) des Schiebedorns aufnehmen kann, oder in einer Hülse endet, die durch lokale Verformung eines Hypotubes erzeugt und mit kontrollierter Kraft direkt auf das Mikrokabel aufgeschoben wird.

4. Einheit nach Anspruch 3, wobei der Aufnahmebehälter die Form eines hohlen Federkegels (72) hat, der schraubend mit dem distalen Ende des Schiebedorns zusammenwirkt.

5. Einheit nach Anspruch 1, wobei:
- sie außerdem eine Dorn zum Schieben des Mikrokabels in den Sondenkörper enthält, und
- der Dorn an seinem distalen Ende in einer Hülse endet, die durch lokale Verformung eines Hypotubes erzeugt wird, und direkt unter kontrollierter Kraft auf das Mikrokabel aufgeschoben werden kann.

6. Einheit nach Anspruch 1, wobei der Sondenkörper und das Mikrokabel so bemessen sind, dass, wenn auf der distalen Seite das Ende des Mikrokabels bündig an der Ausmündung der Öffnung des Sondenkörpers anliegt, auf der proximalen Seite das Mikrokabel dann vollständig in die vom Sondenkörper und vom Verbinder geformte Einheit eingeführt ist.

7. Einheit nach Anspruch 1, wobei das Mikrokabel in der Nähe seines proximalen Endes in der Zone zwischen diesem proximalen Ende und dem Einsatz einen Anschlagring (74) enthält, der fest mit dem Mikrokabel verbunden ist und dessen Durchmesser größer als der Durchmesser der Bohrung des Einsatzes ist, die das Mikrokabel auf der proximalen Seite aufnimmt, um das Gleiten des Mikrokabels im Sondenkörper und folglich die Länge des Teils, der jenseits von dessen distalem Ende austritt, auf eine vorbestimmte maximale Länge zu begrenzen.

8. Einheit nach Anspruch 1, wobei die Sonde aus zwei trennbaren Teilen hergestellt ist, mit (i) einem die eigentliche Sonde bildenden Teil, der auf der proximalen Seite in einem ersten Stecker endet, und (ii) einem einen Verlängerer bildenden Teil, mit auf der distalen Seite einem zweiten Stecker homolog zum ersten Stecker und auf der proximalen Seite dem Verbinder, und wobei der Einsatz auf dem einen Verlängerer bildenden Teil geformt ist.
